# EUROPEAN PATENT SPECIFICATION

(11) **EP 4 333 943 B1**
(45) Date of publication and mention of the grant of the patent: **09.12.2026**
(21) Application number: 22726413.2
(22) Date of filing: 28.04.2022
(51) Int. Cl.: A61M 5/315, A61M 5/20

(54) **SUB-ASSEMBLY FOR A MEDICAMENT DELIVERY**
UNTERBAUGRUPPE FÜR EINE MEDIKAMENTENABGABEVORRICHTUNG
SOUS-ENSEMBLE POUR DISPOSITIF D'ADMINISTRATION DE MÉDICAMENTS

(30) Priority: 07.05.2021 US 202163185398 P; 25.05.2021 EP 21175717
(43) Date of publication of application: 13.03.2024
(73) Proprietor: SHL Medical AG, 6302 Zug (CH)
(72) Inventor: SCOTT, Daniel, Deerfield Beach, Florida 33442 (US); STEFANOV, Slobodan, Deerfield Beach, Florida 33442 (US)
(86) International application number: PCT/EP2022/061352
(87) International publication number: WO 2022/233706

(56) References cited:
- US-A- 6 050 977
- US-A1- 2019 307 959
- US-A1- 2020 046 907

## Description

### TECHNICAL FIELD

The present disclosure generally relates to a component and a sub-assembly for a medicament delivery device.

### BACKGROUND

Medicament delivery devices such as pen type manual injectors or auto-injectors are generally known for the self-administration of a medicament by patients without formal medical training. For example, patients suffering from diabetes may require repeated injections of insulin, or patients may require regular injections of other types of medicaments, such as a growth hormone. For facilitating the patients to correctly perform a self-administration, one or more indications for indicating one or more stages of an operation of a medicament delivery device is important.

Further, for some types of medicaments, there is a desire to eject the medicament at a substantially constant force. However, certain existing devices for ejecting the medicament at a substantially constant force are complex and expensive to manufacture. There is a desire to reduce the cost of manufacturing automatic injection devices while maintaining the reliability of the injection device to eject the medicament at a substantially constant force.

It has been appreciated that solutions for providing one or more indications regarding one or more stages of a medicament delivery operation and/ or for providing a mechanism of keeping the medicament being delivered under a substantially constant force could be advantageous. Examples of the prior art can be found in US6050977A, US2019/307959A1 and US2020/046907A1.

### SUMMARY

The invention is defined by the appended claims, to which reference should now be made.

In the present disclosure, when the term "distal direction" is used, this refers to the direction pointing away from the dose delivery site during use of the medicament delivery device. When the term "distal part/end" is used, this refers to the part/end of the delivery device, or the parts/ends of the members thereof, which under use of the medicament delivery device is/are located furthest away from the dose delivery site. Correspondingly, when the term "proximal direction" is used, this refers to the direction pointing towards the dose delivery site during use of the medicament delivery device. When the term "proximal part/end" is used, this refers to the part/end of the delivery device, or the parts/ends of the members thereof, which under use of the medicament delivery device is/are located closest to the dose delivery site.

Further, the term "longitudinal", "longitudinally", "axially" or "axial" refer to a direction extending from the proximal end to the distal end, typically along the device or components thereof in the direction of the longest extension of the device and/or component.

Similarly, the terms "transverse", "transversal" and "transversally" refer to a direction generally perpendicular to the longitudinal direction.

Further, the terms "circumference", "circumferential", or "circumferentially" refer to a circumference or a circumferential direction relative to an axis, typically a central axis extending in the direction of the longest extension of the device and/or component. Similarly, "radial" or "radially" refer to a direction extending radially relative to the axis, and "rotation", "rotational" and "rotationally" refer to rotation relative to the axis.

### BRIEF DESCRIPTION OF THE DRAWINGS

Fig. 1 schematically shows a perspective view of components of a medicament delivery device with a plunger rod of the invention.
Figs 2A-B show a side view and a cross-section view of the plunger rod of Fig. 1.
Fig. 3A shows a perspective view of a drive assembly of the medicament delivery device of Fig. 1.
Figs 3B-D show cross-section views of the drive assembly of Fig. 3A.
Fig. 4A shows a perspective view of the drive assembly of Fig. 3A being used with the plunger rod of Fig. 1 and a button.
Fig. 4B shows a cross-section view of Fig. 4A.
Fig. 5 shows a perspective view of a housing of the medicament delivery device of Fig. 1.
Figs 6A-B show cross-section views of the plunger rod of Fig. 1 in different embodiments.
Figs 7A-B show side views of the plunger rod of Fig. 1 used with the drive assembly of Fig. 3A.
Fig. 8 shows a perspective view of a proximal portion of a guide assembly of the drive assembly of Fig. 3A.
Fig. 9A shows a perspective view of the medicament delivery device of Fig. 1.
Fig. 9B shows a cross-section view of the medicament delivery device of Fig. 9A.
Fig. 9C shows a cross-section view of another embodiment of the medicament delivery device of Fig. 9A.
Figs 10-11 show cross-section views of a sub-assembly two different examples.
Fig. 12 shows a cross-section view of a medicament delivery device using the sub-assembly.
Fig. 13 shows a perspective view of the sub-assembly.
Figs 14-15 show cross-section views of a sub-assembly of Fig. 13 in two different examples.

### DETAILED DESCRIPTION

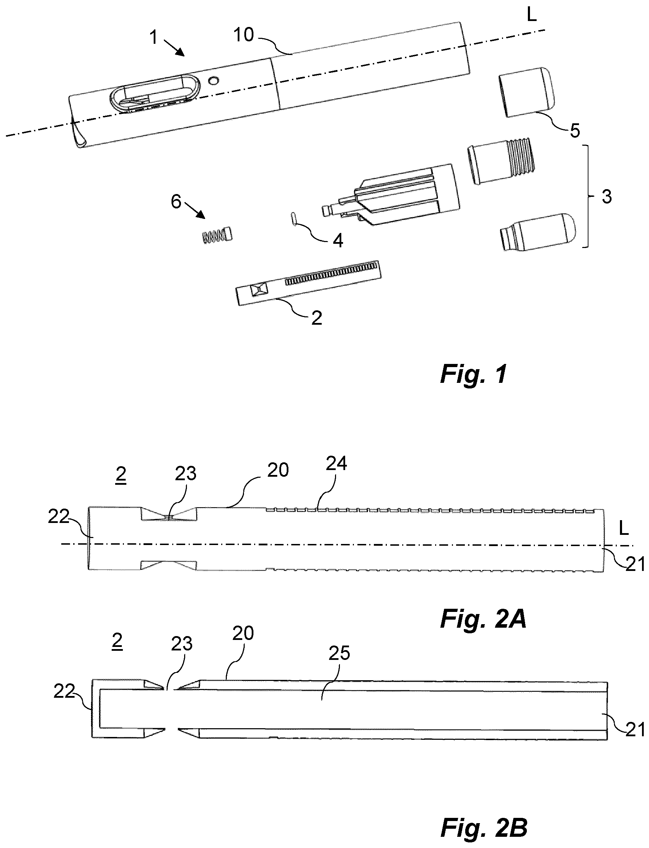

Figs 1-9C illustrate a plunger rod 2 for a medicament delivery device 1. The plunger rod 2 comprises a tubular body 20 and a whistle orifice 23. The tubular body 20 extends along a longitudinal axis L between a closed end 22 and an open end 21. The tubular body 20 comprises a tubular wall defined an interior space of the tubular body 20, as shown in Fig. 2B. The whistle orifice 23 of the plunger rod is on the tubular wall of the tubular body 20, and positioned between the open end 21 of the tubular body 20 and the closed end 22 of the tubular body 20.

The plunger rod 2 is configured to connect with a powerpack of the medicament delivery device 1. Usually, a powerpack of the medicament delivery device comprises a drive assembly and a user-accessible trigger which is a component, or an assembly, connected to the drive assembly that can be moved manually by the user. The drive assembly of the medicament delivery device comprises an energy source, e.g. a spring, gas canister or motor, and will connect to the plunger rod 2.

The movement of the trigger by the user will cause the drive assembly to transfer stored energy from the energy source to the plunger rod 2 so that the plunger rod 2 can actuate a medicament contained within the medicament delivery device and expel the medicament to the user. The trigger may be a button, a delivery member guard or an assembly comprising a button or a delivery member guard.

The tubular body 20 of the plunger rod 2 of the invention is configured to allow an airflow flowing within an interior chamber 25 of the tubular body 20 during a medicament delivery operation. When the airflow passes through the whistle orifice 23, an audible indication for the user can therefore be generated.

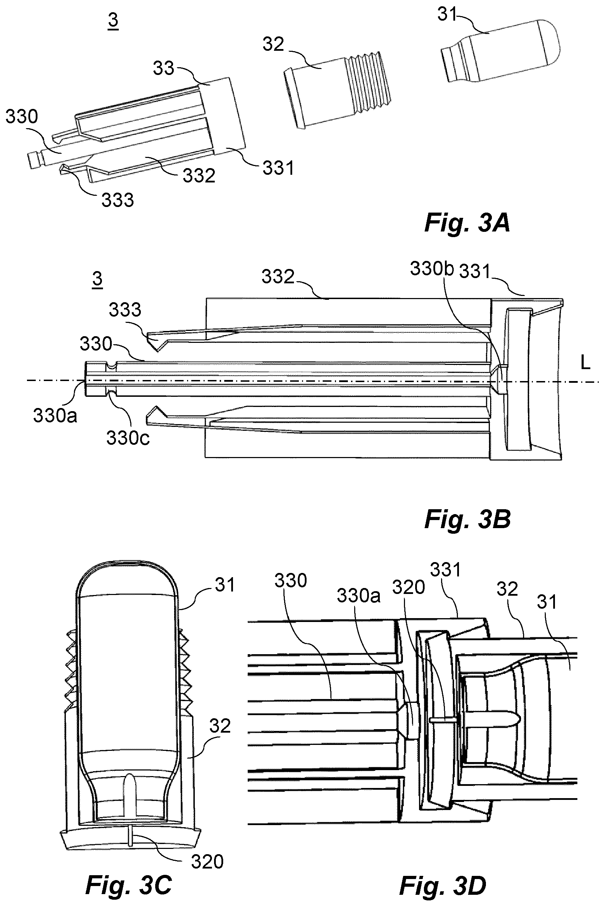

In one example, the plunger rod 2 is connected to a drive assembly 3 with a gas canister 31. For example, as shown in Fig. 3A, the drive assembly 3 comprises a gas canister storing pressurized gas, e.g. liquefied gas or pressurized pneumatic gas, a valve unit 32 and a guide assembly 33.

In an example, as shown in Fig. 1, the plunger rod 2 and the drive assembly 3 are used in a sub-assembly for a medicament delivery device 1. The sub-assembly comprises a housing 10 extending along the longitudinal axis L between a proximal end and a distal end. The plunger rod 2 and the drive assembly are both received within the housing 10. The plunger rod 2 is axially movable relative to the housing 10. The housing 10 can also act as the housing of the medicament delivery device 1.

The valve unit 32 is configured to release the gas from the gas canister. The valve unit 32 comprises an air inlet and an air outlet. Preferably, a valve stem 320 is extending between the air inlet and the air outlet, as shown in Fig. 3C. The air inlet is movable, e.g. axially and/ or rotationally, relative to the gas canister. The relative movement between the air inlet of the valve unit 32 is configured to release the gas from the gas canister so that the gas can flow out from the gas canister from the air inlet of the valve unit 32 to the ait outlet of the valve unit 32. The valve unit 32 may comprise a holding body to receive the gas canister 31 so that the gas canister and valve unit 32 can be attached to a medicament delivery device via the holding body of the valve unit 32.

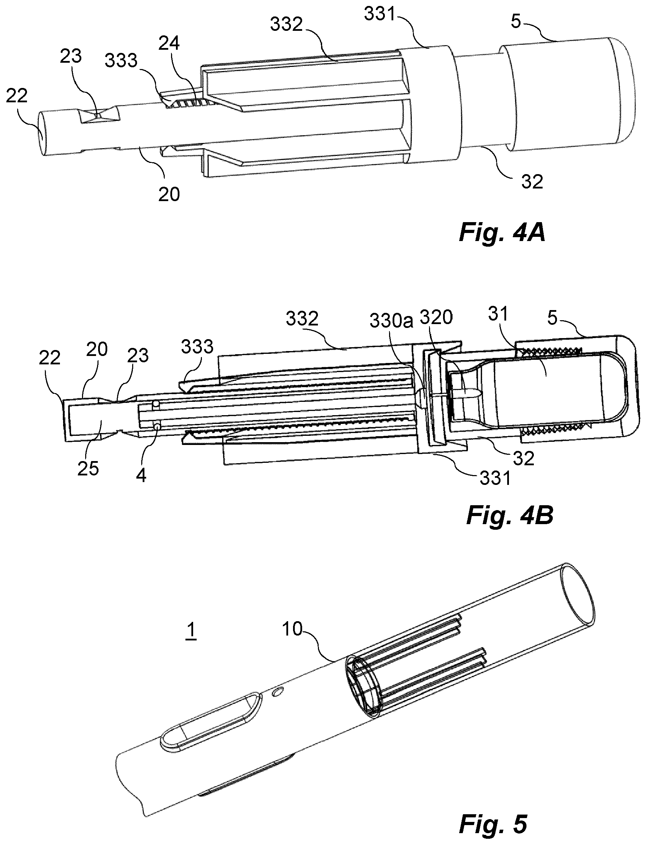

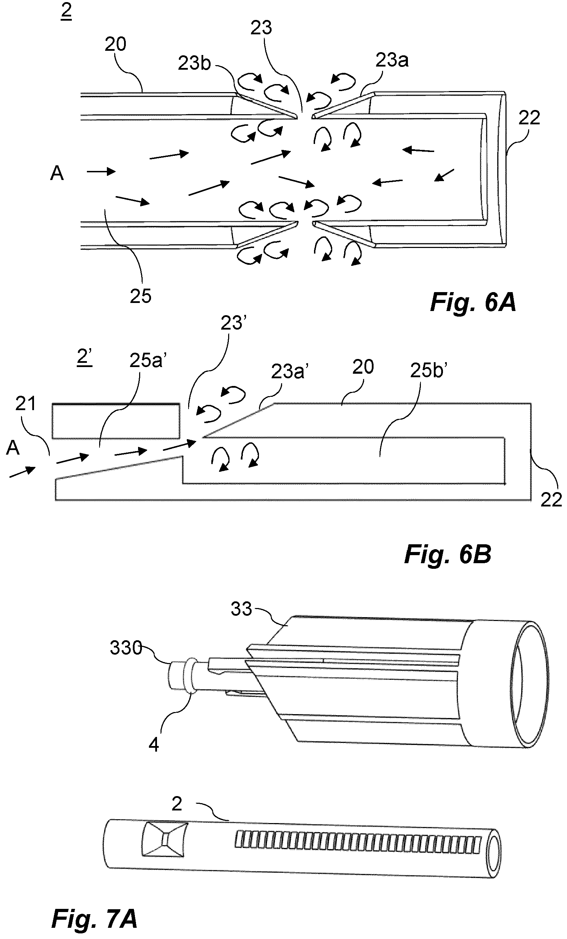

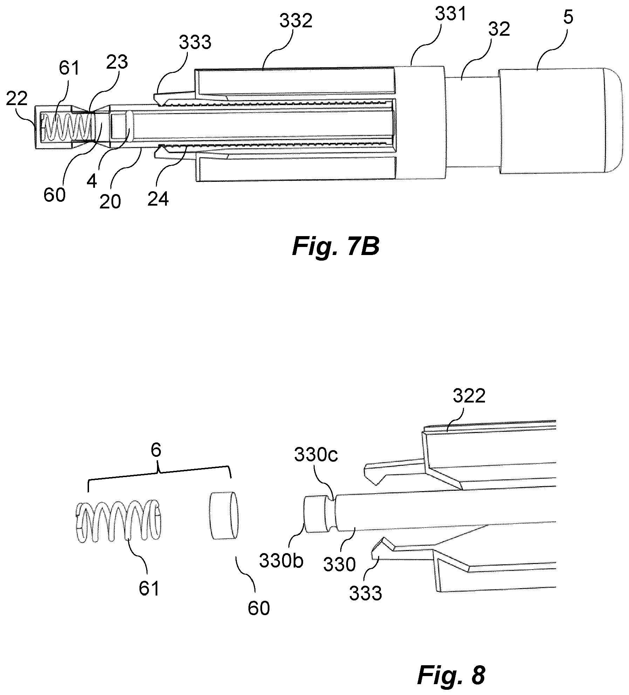

The guide assembly 33 is configured to guide the released gas from the gas canister into the interior chamber 25 of the tubular body 20 of the plunger rod 2. As shown in Fig. 3B, the guide assembly 33 comprises a sleeve 330 extending along the longitudinal axis L axis between a first opening 330a and a second opening 330b. The first opening 330a of the sleeve 330 is connected to the open end 21 of the tubular body 20 of the plunger rod 2; and the second opening 330b of the sleeve 330 is gas-tightly connected to the air outlet of the valve 32, as shown in Fig. 3D. In one example, the sleeve 330 is positioned at least partially within the interior chamber 25 of the plunger rod 2, as shown in Fig. 4B. The guide assembly 33 optionally comprises a base 331 for receiving at least a part of the valve unit 32 together with the gas canister 31. The guide assembly 33 also may also comprise an attachment 332 configured to attach the guide assembly to a medicament delivery device. The attachment 332 may comprise a flexible arm 333 protruding towards the direction that the first opening 330a is located. Further, the sleeve 330 may comprise a recess 330c on its outer surface and positioned between the first opening 330a and the second opening 330b. The flexible arm 333 will be explained in detail later. In a preferred example, the drive assembly is connected with an elastic ring 4., The elastic ring 4 can attached to the recess 330c of the sleeve 330, as shown in Figs. 7A-B. In the first aspect of the invention, the elastic ring 4 is configured to gas-tightly seal between the outer surface of the sleeve 330 and an inner surface of the tubular body 20 of the plunger rod 2. Therefore, the gas flowing out from the first opening 330a of the sleeve 330 into the interior chamber 25 of the tubular body 20 of the plunger rod 2 can only flow towards the closed end 22 of the plunger rod 2.

The user-accessible trigger for the medicament delivery device can be arranged to connect to the valve or the gas canister or the guide assembly. For example, the user-accessible trigger is a button 5 attached to the gas canister 31, as shown in Figs 4A-4B. In this example, the guide assembly 33 (also the sleeve 330) and the valve unit 32 are axially fixed to the housing 10, the user may axially press on the button and therefore cause the gas canister to move axially relative to the air inlet of the valve unit 32. The axial movement between the air inlet of the valve unit 32 and the gas canister 31 releases the gas from the gas canister 31 so that the gas will flow into the interior chamber 25 of the plunger rod 2 through the sleeve 330 of the guide assembly 33. Once the gas flows A within the interior chamber 25 of the plunger rod 2, as shown in Figs 6A-6B, some swirls will be generated around the whistle orifice 23; 23'; an audible indication can be therefore created. The user can thus receive the audible indication as a sign that a medicament delivery operation is in progress. The whistle orifice 23; 23' also serves as a pressure releasing orifice to release the gas within the interior chamber 25 of the plunger rod 2. Therefore, an air pressure within the interior chamber 25 of the plunger rod 2 will not keep increasing. Damage of the medicament container caused by a significant force from the plunger rod can therefore be prevented or minimised.

It should be noted that, alternatively, the trigger can connect to the valve unit and the delivery member guard, in the example, the guide assembly and the gas canister are attached to the housing of the medicament delivery device. When the user presses the delivery member guard on the medicament delivery site, the valve unit can be moved by the delivery member guard relative to the gas canister. In this example, the guide unit (also the sleeve) is axially fixed to the housing. Alternatively, the guide assembly can be connected to the medicament delivery member guard, so that the guide assembly is axially movable relative to the housing together with the medicament delivery member guard. In this example, the gas canister is axially fixed to the housing of the medicament delivery device. The valve unit can be moved by the guide assembly together with the movement of the delivery member guard by the user.

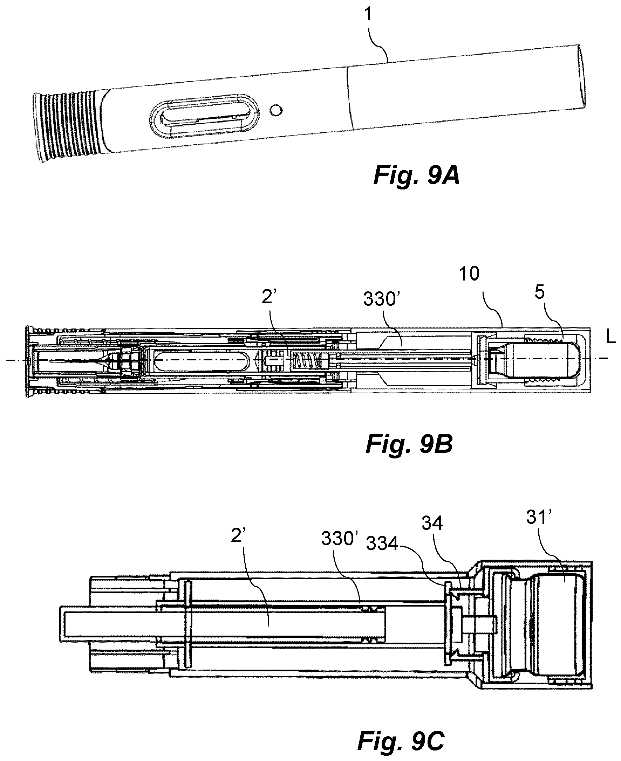

Furthermore, the drive assembly 3 may comprise an arrangement for retaining the flow communication between the gas canister 31 and the sleeve 330; 330'. As shown in Fig. 9C, the sleeve 330' may comprise a flange 334, and the drive assembly 3 may comprise a clip 34. In this example, the movement of the trigger will cause the relative movement between the clip 34 and the sleeve 330'; therefore, the clip 34 will clip on the flange 334 of the sleeve 330'. In this example, the clip 34 can be fixedly connected to the gas canister 31. Therefore, when the gas canister 31 moves relative to the valve stem 332 upon the movement of the trigger, the clip 34 will clip on the flange 334 of the sleeve and therefore retain the gas canister 31 in a position that the contained keeps releasing from the valve stem. In this example, the user doesn't need to keep pressing on the trigger during the whole medicament delivery operation.

In another preferred example, the plunger rod 2 comprises a blocker unit arranged within the tubular body 20 of the plunger rod 2. The blocker unit is movable along the longitudinal axis L between a blocked position where the blocker unit is radially aligned with the whistle orifice relative to the longitudinal axis L and a release position where the blocker unit is radially misaligned with the whistle orifice 23 relative to the longitudinal axis L. In one example, the blocker unit 6 comprises a blocker body 60 and a biasing member 61, as shown in Fig. 8. The biasing member 61 is positioned between the closed end 22 of the plunger rod 2 and the blocker body 60 along the longitudinal axis L. The biasing member 61 can be a compression spring or a flexible arm. When the biasing member 61 is in its relaxed configuration, the biasing member 61 is configured to support the blocker body 60 to radially align with the whistle orifice 23 of the plunger rod 2; therefore gas that flows within the interior chamber 25 of the plunger rod 2 cannot flow out from the whistle chamber 23. In a preferred example, the blocker body 60 is gas-tightly sealed onto the inner surface of the tubular body 20 of the plunger rod 2. If the gas that flows within the interior chamber 25 of the plunger rod 2 cannot flow out from the whistle chamber 23 and the gas within the gas canister is released by the valve unit 32, the pressure within the interior chamber 25 of the plunger rod 2 will keep increasing. Once the pressure within the interior chamber 25 of the plunger rod 2 reaches a certain level that is enough to compress the biasing member 61 of the blocker unit 6, the blocker body 60 will move under the pressure within the interior chamber 25 of the plunger rod 2 into its release position, so that the blocker body 60 will radially misalign with the whistle orifice 23 relative to the longitudinal axis L. When the blocker body 60 is radially misaligned with the whistle orifice 23 relative to the longitudinal axis L, the gas flowing within the interior chamber 25 of the plunger rod 2 can flow out from the whistle orifice 23. There are at least two main advantages that can be provided by the arrangement of the blocker unit 6. Firstly, the blocker unit 6 can keep the plunger rod 2 moving constantly. For example, the force figure of the biasing member 61 can be chosen depending on the viscosity of the medicament contained within the medicament delivery device with the plunger rod 2 of this invention. Therefore, if the released gas power is not enough to expel the medicament, the blocker body 60 is in the blocked position, the whistle orifice 23 will be sealed by the blocker body 60 so that the pressure within the interior chamber 25 of the plunger rod 2 will increase until the accumulated pressure is enough to firstly compress the biasing member 60 and expel the medicament. When released gas power is high enough to expel the medicament, the blocker body 60 will be moved to the release position under the flow pressure within the interior chamber 25 of the plunger rod 2, the whistle orifice 23 will open due to the misalignment of the blocker body 60 in the radial direction relative to the longitudinal axis L. Secondly, the user will get a more precise progress indication for the medicament delivery operation. Because the user can only hear the whistle sound (the audible medicament delivery indication) when the whistle orifice 23 is no longer blocked by the blocker body 60, meaning the flow pressure within the interior chamber 25 of the plunger rod 2 is high enough to compress the biasing member 61 and then move the plunger rod 2. Usually, in a disposable medicament delivery device, the gas canister will be selected so that only contains an amount of gas required to complete a single medicament delivery operation. Therefore, when the medicament delivery operation is finished, the gas canister cannot supply enough gas flow to keep the flow pressure within the interior chamber 25 of the plunger rod high enough to compress the biasing member 61 and keep the blocker body 60 in its release position; so that when the medicament delivery operation is finished, the biasing member 61 moves the blocker body 60 back to the blocked position. The whistle orifice 23 will therefore be blocked by the blocker body 60 at the end of the medicament delivery operation. Once the whistle orifice 23 is blocked by the blocker body 60, there is no longer a whistling sound being generated, the user, therefore, is indicated (by the whistle sound stopping) that the medicament delivery operation is completed.

It should be noted that the plunger rod 2 with the whistle orifice 23; 23', as provided by the invention, is suitable for connecting with any medicament delivery device power pack that is able to generate an airflow flowing into the tubular body 20 of the plunger rod 2. For example, instead of a gas canister and the valve, the drive assembly may comprise an airflow generating unit, e.g. an air pump, that is connected to the sleeve. The airflow that is generated by the air pump can therefore enter into the tubular body 20 of the plunger rod 2. The air pump may connect to a motor so that the motor is configured to output a force that presses on the air pump. Alternatively, the air pump may be accessible by the user, so that the user is able to manually press on the air pump to generate the airflow and thus actuate the plunger rod in the proximal direction.

Furthermore, in another example, instead of a gas canister or an airflow generating unit, the drive assembly may comprise a spring or motor as an energy source. In this example, the open end of the plunger rod can be arranged to aim at the medicament container, and the closed end of the plunger rod can be connected to a energy source, for example, a compression spring. In this example, the open end of the plunger rod can be spaced apart from a distal seal of the medicament container, e.g. a stopper, at the beginning. When the energy source moves the plunger rod in the proximal direction, the plunger rod will squeeze the air between the open end of the plunger rod and the distal end of the distal seal of the medicament container before the plunger rod reaches to the distal seal of the medicament container . The squeezed air will escape at least partially from the whistle orifice, the user thus can hear a whistling sound as a start indication of the medicament delivery operation. In a preferred example, the plunger rod may be dimensioned with a diameter that is a snug fit with a diameter of the distal end of the medicament container; alternatively, an indication tube can be arranged between the medicament container and the plunger rod, so that the plunger rod needs to pass the indication tube first before entering into the medicament container or reaching to the distal seal of the medicament container. The plunger rod will then be dimensioned with a diameter that is a snug fit with a diameter of the indication tube.

The whistle orifice 23; 23' can be formed in a different geometry, for example, the tubular body 20 of the plunger rod 2 comprises at least one inclined surface 23a; 23a' angled relative to the longitudinal axis L and extending from the whistle orifice 23 in the direction of the longitudinal axis L. In another preferred example, as shown in Fig. 6A, the tubular body 20 of the plunger rod 2 comprises a first inclined surface 23a and a second inclined surface 23b. The first inclined surface 23a is angled relative to the longitudinal axis L and extending from the whistle orifice 23 towards the open end 21 of the tubular body 20. The second inclined surface 23b is angled relative to the longitudinal axis L and extending from the whistle orifice 23 towards the closed end 22 of the tubular body 20. Alternatively, in another preferred example, as shown in Fig. 6B, the tubular body 20 of the plunger rod 2 comprises a first inner cylindrical chamber 25a' and a second inner cylindrical chamber 25b'. The first inner cylindrical chamber 25a' is arranged between the open end 21' of the tubular body 20 and the whistle orifice 23'. The second cylindrical chamber 25b' is arranged between the closed end 22 of the tubular body 20 and the whistle orifice 23'. The first inner cylindrical chamber 25a' has a diameter smaller than a diameter of the second inner cylindrical chamber 25b'. In another example, the plunger rod 2 comprises two whistle orifices 23 on the tubular wall of the tubular body 20. The two orifices 23 are symmetrically arranged relative to the longitudinal axis L and arranged between the open end 21 of the tubular body 20 and the closed end 22 of the tubular body 20.

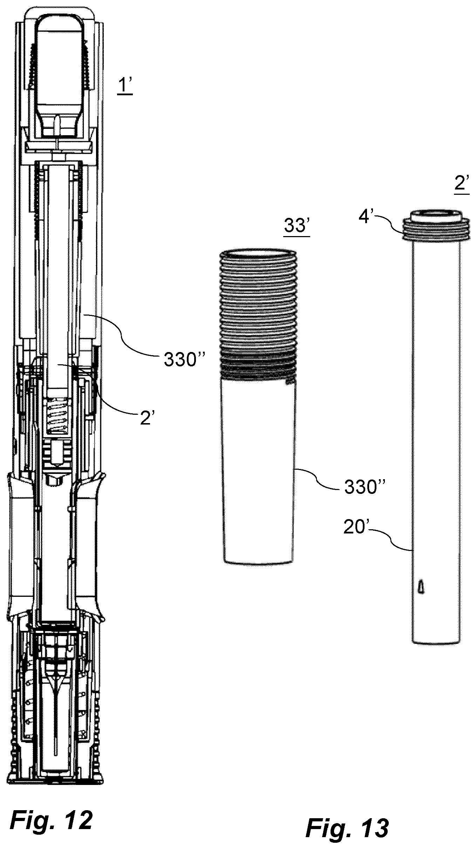

Figs 9A-15 illustrate an unclaimed sub-assembly for a medicament delivery device 1; 1'. The sub-assembly comprises a plunger rod 2; 2' extending along the longitudinal axis L between a proximal end and a distal end; a sleeve 330; 330'; 330"; 330‴ extending along the longitudinal axis L between a proximal end and a distal end. The sleeve 330; 330'; 330"; 330‴ is coaxially to the plunger rod 2; 2' relative to the longitudinal axis L and connected to the plunger rod 2; 2' relative to the longitudinal axis L. The sub-assembly comprises the elastic ring 4; 4' positioned between the plunger rod 2; 2' and the sleeve 330; 330'; 330"; 330‴. The elastic ring 4; 4' is configured to surround the at least one of the plunger rod 2; 2'and the sleeve 330; 330'; 330"; 330"', as shown in Fig. 7A and Fig. 13. The plunger rod 2; 2' is axially movable along the longitudinal axis L relative to the sleeve 330; 330'; 330"; 330"'. The plunger rod 2; 2' comprises a tubular body 20; 20'; 20"; 20‴. One of the sleeve 330; 330'; 330"; 330‴and the tubular body 20; 20'; 20"; 20‴ of the plunger rod 2; 2' is dimensioned with a diameter that monotonically varies along the longitudinal axis L from the proximal end towards the distal end. The elastic ring 4; 4' is attached to the other one of the sleeve 330; 330'; 330"; 330‴ and the plunger rod 2; 2'.

The sub-assembly can keep the plunger rod, when subjected to a force from the energy source, moving in the proximal direction constantly. For example, the arrangement as defined above can keep the flow pressure within the tubular body 20; 20'; 20"; 20" of the plunger rod 2, and therefore keep the force applied on the plunger rod 2 for moving the plunger rod 2 in the proximal direction of the medicament delivery device constant. In this example, the sub-assembly is preferably used with a gas power medicament delivery device with a gas canister as mentioned above, or connected to an airflow generating unit, e.g. an air pump. The sleeve 330; 330'; 330"; 330‴ can be arranged to connect with a gas canister as the energy source of the medicament delivery device 1.

An exemplified connection between the sleeve 330; 330' can be arranged as described in the first aspect mentioned above, namely, the sleeve 330; 330' is a part of the guide assembly 33 and is positioned within the tubular body 20; 20" of the plunger rod 2, so that the gas released from the gas canister can flow into the tubular body 20; 20" of the plunger rod 2 and actuate the plunger rod 2 to move in the proximal direction of the medicament delivery device 1. In this example, similar to the first aspect mentioned above, the tubular body 20; 20" of the plunger rod 2' comprises the closed end at the proximal end of the plunger rod 2' and the open end at the distal end of the plunger rod 2. In the second aspect of the invention, the plunger rod 2 may optionally comprise the whistle orifice as mentioned above; or the indication of the medicament delivery operation can be provided by the flexible arm 333 on the guide assembly 33 and a set of teeth 24 on the outer surface of the tubular body 20; 20" of the plunger rod 2, as shown in Fig. 4A.

Alternatively, the plunger rod 2 can be positioned at least partially within the sleeve 330"; 330‴, as shown in Figs 12-15. In this example, the sleeve 330"; 330‴ may connect to a valve for releasing the gas from a gas canister. Similar to the trigger arrangement as described above, the sleeve 330"; 330‴ and the valve can be attached to the housing of the medicament delivery device 1'. The gas canister can be connected to a user-accessible button. Alternatively, the trigger can connect to the valve unit and the delivery member guard, in the example, the guide assembly and the gas canister are attached to the housing of the medicament delivery device. When the user presses the delivery member guard on the medicament delivery site, the valve unit can be moved by the delivery member guard relative to the gas canister. Alternatively, the guide unit can be connected to the medicament delivery member guard, in this example, the gas canister is attached to the housing of the medicament delivery device. The valve unit can be moved by the guide unit together with the movement of the delivery member guard by the user. In this example, the tubular body 20'; 20‴ of the plunger rod 2 comprises the closed end at the proximal end of the plunger rod 2 and the open end at the distal end of the plunger rod 2. When the trigger is moved by the user to trigger a medicament delivery operation, the released gas will flow into the tubular body 20'; 20‴ of the plunger rod 2 and actuate the plunger rod 2 to move in the proximal direction of the medicament delivery device 1'.

Usually, the amount of gas released from the gas canister in a certain period will also be dependent on the flow pressure within the gas canister. For example, when a new gas canister starts to release gas, the amount of gas released from the gas canister in a certain period will be high; so that if the plunger rod 2 connects to the gas canister, the flow pressure within the tubular body 20; 20'; 20"; 20‴ of the plunger rod 2 can also be high at the beginning. However, when the gas canister continuously releases the gas for a while, the amount of gas released from the gas canister in the certain period will decrease, due to the flow pressure dropped within the gas canister. Therefore, if the plunger rod 2 connects to the gas canister, the flow pressure within the tubular body 20; 20'; 20"; 20‴ of the plunger rod 2 will also drop when the plunger rod 2 has been moved for a while in the proximal direction of the medicament delivery device.

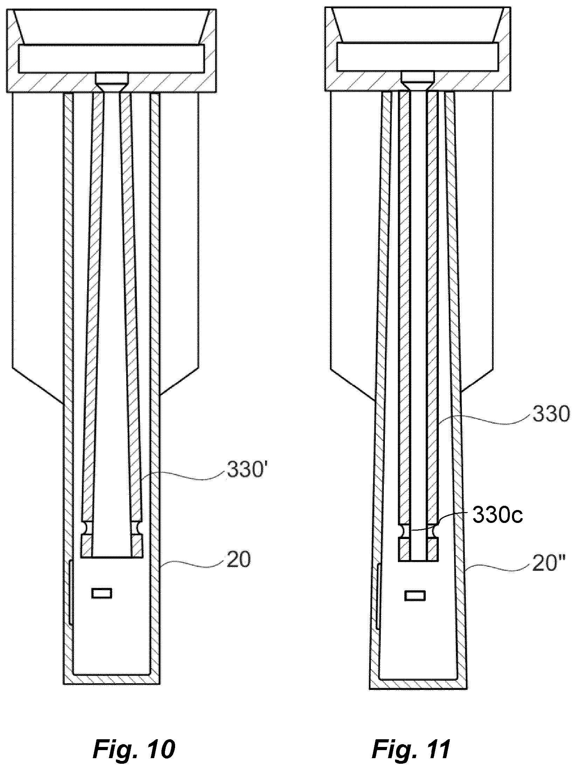

Fig. 10 illustrates one example of the sub-assembly.

In this example, the sleeve 330' is arranged within the tubular body 20 of the plunger rod 2. The sleeve 330' is dimensioned with an outer diameter increased gradually from the distal end of the sleeve towards the proximal end of the sleeve 330'. In this example, the elastic ring (not shown in the Fig. 10) is attached on an inner surface of the tubular body 20 of the plunger rod 2 and is fixed to the plunger rod 2 in the direction of the longitudinal axis L; for example, the inner surface of the plunger rod may comprise an annular groove for receiving the elastic ring. The elastic ring can be attached at the distal end of the plunger rod 2, so that when the plunger rod 2 is actuated by the released gas from the gas canister, as the energy source of the medicament delivery device 1, the plunger rod 2 and the elastic ring will axially move together relative to the sleeve 330' towards the proximal direction of the housing of the medicament delivery device. Because the outer diameter of the sleeve 330' increases gradually from the distal end of the sleeve towards the proximal end of the sleeve 330', a gap between the elastic ring and the outer surface of the sleeve 330' is greater when the plunger rod 2 starts to move relative to the sleeve 330' in the proximal direction of the housing of the medicament delivery device. The gap between the elastic ring and the outer surface of the sleeve 330' will then become narrower and narrower when the plunger rod 2 together with the elastic ring moves closer and closer to the proximal end of the sleeve 330'. As mentioned above, the released gas will flow into the tubular body 20 of the plunger rod 2 from the sleeve 330'; when the gas canister has been initially triggered to release the contained gas, the gap between the elastic ring and the sleeve 330' causes part of the released gas leaks out; therefore the flow pressure within the tubular body 20 of the plunger rod can only increase to a certain level. When the gas canister has been triggered for a while, meaning the amount of the released gas will be dropped due to the decreased pressure within the gas canister, the plunger rod 2 now together with the elastic ring is closer to the proximal end of the sleeve 330; the gap between the elastic ring and the sleeve 330' is also narrower, or the elastic ring may gas-tightly seal between the plunger rod 2 and the sleeve 330'; therefore, the gas leakage from the gap between the elastic ring and the sleeve 330' will decrease or even stop. The flow pressure within the tubular body 20 of the plunger rod 330' can be therefore maintained in the certain level instead of dropping together due to the decreasing amount of the released gas.

Instead of arranging the sleeve 330' with the outer diameter increased gradually from the distal end of the sleeve 330' towards the proximal end of the sleeve 330', the tubular body 20" of the plunger rod 2 can be dimensioned with an inner diameter increased gradually from the distal end of the tubular body 20" of the plunger rod 2 towards the proximal end of the tubular body 20" of the plunger rod 2, as shown in Fig. 11. In this example, the elastic ring is fixed at the proximal end of the sleeve 330, like the elastic ring can be fixed in the recess 330c of the sleeve 330 as mentioned above.

Similar to the example shown in Fig. 10, when the plunger rod 2 moves relative to the sleeve in the proximal direction of the medicament delivery device, the radial gap between the plunger rod 2 and the elastic ring will be narrower; the leakage of the gas flow from the gap can be thus decreased or even eliminated. The flow pressure within the tubular body 20" of the plunger rod 2 can be therefore maintained at a certain level.

In another example that the plunger rod 2 is positioned at least partially within the sleeve 330"; 330"', as shown in Figs 12-15. Similar to the description above, at least one of tubular body 20'; 20‴ of the plunger rod 2' and the sleeve 330"; 330‴ should be dimensioned with a diameter gradually increasing, opposite to the above examples, from the proximal end of the at least one of tubular body 20'; 20‴ of the plunger rod 2' and the sleeve 330"; 330‴ to the distal end of the at least one of tubular body 20'; 20‴ of the plunger rod 2' and the sleeve 330"; 330"'.

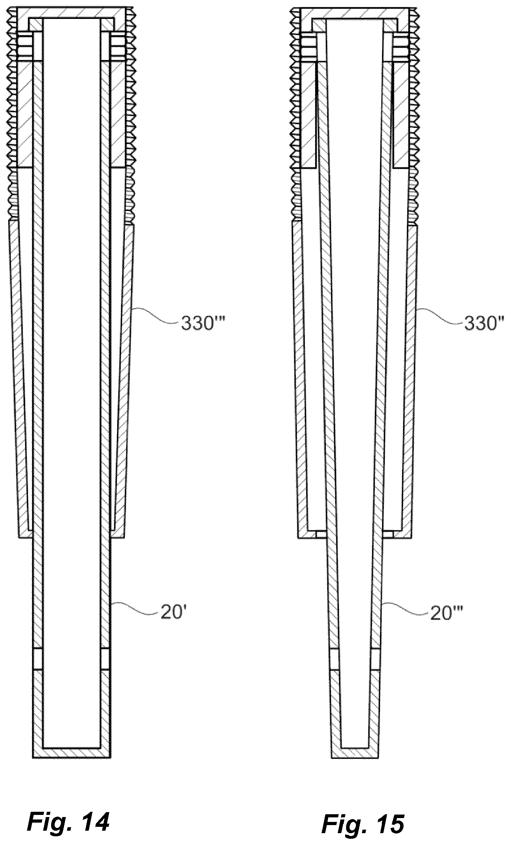

For example, Fig. 14 shows the plunger rod 2' is received within the sleeve 330", the elastic ring 4' has been attached to an outer surface, preferably the distal end of the plunger rod 2', as shown in Fig. 13. In this example, the sleeve 330" is dimensioned with an outer diameter increased gradually from the proximal end of the sleeve 330" towards the distal end of the sleeve 330". Alternatively, the tubular body 20‴ the plunger rod 2' is dimensioned with an outer diameter increased gradually from the proximal end of the plunger rod 2' towards the distal end of the plunger rod 2', as shown in Fig. 15. In the latter example, the elastic ring is attached on an inner surface of the sleeve 330" and at the proximal end of the sleeve 330". Therefore, when the plunger rod 2' moves relative to the sleeve 330"; 330‴ in the proximal direction of the medicament delivery device, the radial gap between the plunger rod 2' and the elastic ring will be narrower; the leakage of the gas flow from the gap can be thus decreased or even eliminated. The flow pressure within the tubular body 20'; 20‴ of the plunger rod 2' can be therefore maintained at a certain level.

It should be noted that, whether the elastic ring should eventually gas-tightly seal between the plunger rod 2; 2' and the sleeve 330; 330'; 330"; 330‴, and/ or where the elastic ring should start to gas-tightly seal between the plunger rod 2; 2' and the sleeve 330; 330'; 330"; 330‴, and also what exact flow pressure level should be kept within the tubular body 20; 20'; 20‴; 20‴ of the plunger rod 2; 2' should be dependent on the design of the medicament delivery device, e.g. dependent on the volume of the medicament or the viscosity of the medicament. Furthermore, in the second aspect of the invention, since the flow pressure within the tubular body 20; 20'; 20‴; 20‴ of the plunger rod 2; 2' is regulated by the amount of the gas leakage from the radial gap between the elastic ring and the plunger rod; the sub-assembly preferably does not comprise the block unit 6.

It should be noted that the above explanation refers to a gas or airflow energy source as an example; however, the sub-assembly also be used with other suitable energy sources, for example, a spring. As mentioned above, one of the sleeve and the tubular body of the plunger rod is dimensioned with a diameter that monotonically varies along the longitudinal axis L from the proximal end towards the distal end. The elastic ring is attached to the other one of the sleeve and the plunger rod. However, if the energy source is, for example, a compression spring, the sub-assembly may be modified so that the elastic ring tightly seals the gap between the sleeve and the plunger rod at the beginning of the medicament delivery operation, and becomes a loose seal or even stays away from one of the plunger rod or the sleeve later on due to the monotonically varying diameter. Therefore, the elastic ring can generate relatively significant friction between the sleeve and the plunger rod at the beginning to act as a brake for the plunger rod and gradually reduces the resistance later on.

The invention is defined by the appended claims.

## Claims

1. A sub-assembly for a medicament delivery device, the sub-assembly comprising:
- a plunger rod (2) comprising:
∘ a tubular body (20) extending along a longitudinal axis (L) between a closed end (22) and an open end (21); and
∘ a whistle orifice (23; 23') on a tubular wall of the tubular body (20); wherein the whistle orifice (23; 23') is positioned between the open end (21) of the tubular body (20) and the closed end (22) of the tubular body (20); wherein the tubular body (20) of the plunger rod (2) comprises at least one inclined surface (23a; 23a') angled relative to the longitudinal axis (L) and extending from the whistle orifice (23; 23') in the direction of the longitudinal axis (L);
- a power pack able to generate an airflow flowing into the tubular body (20).

2. The sub-assembly according to claim 1, wherein the tubular body (20) of the plunger rod (2) comprises a first inclined surfaces (23a) and a second inclined surface (23b); wherein the first inclined surface (23a) is angled relative to the longitudinal axis (L) and extending from the whistle orifice (23) towards the open end (21) of the tubular body (20); and wherein the second inclined surface (23b) is angled relative to the longitudinal axis (L) and extending from the whistle orifice (23) towards the closed end (22) of the tubular body (20).

3. The sub-assembly according to any one of the preceding claims, comprising a blocker unit (6) arranged within the tubular body (20) of the plunger rod (2); wherein the blocker unit (6) is movable along the longitudinal axis (L) between a blocked position where the blocker unit (6) is radially aligned with the whistle orifice (23) relative to the longitudinal axis (L) and a release position where the blocker unit (6) is radially misaligned with the whistle orifice (23) relative to the longitudinal axis (L).

4. The sub-assembly according to claim 3, wherein the blocker unit (6) comprises a blocker body (60) and a biasing member (61), and wherein the biasing member (61) is positioned between the closed end (22) of the tubular body (20) and the blocker body (60).

5. The sub-assembly according to any one of claims 1-4; wherein the sub-assembly comprises
- a housing (10) extending along a longitudinal axis (L) between a proximal end and a distal end; and
- a drive assembly (3) configured to be connected to a trigger of the medicament delivery device; the drive assembly and the trigger being part of the power pack;
wherein the drive assembly (3) is connected to the housing and connected to the plunger rod (2).

6. The sub-assembly according to claim 5, wherein the drive assembly (3) comprises a gas canister (31), a valve unit (32) and a sleeve (330); wherein the valve unit (32) comprises a valve; wherein the valve comprises an air inlet and an air outlet; wherein the air inlet connects to the gas canister (31);wherein the sleeve (330) extends along the longitudinal axis (L) between a first opening (330a) and a second opening (330b); wherein the first opening (330a) of the sleeve (330) is connected to the open end (21) of the tubular body (20) of the plunger rod (2); wherein the second opening (330b) of the sleeve (330) is gas-tightly connected to the air outlet of the valve (320); and wherein the gas canister (31) is axially movable relative to the air inlet of the valve (320) between a gas release position and a gas sealed position.

7. The sub-assembly according to claim 6, wherein the sub-assembly comprises an elastic ring (4) arranged between the tubular body (20) of the plunger rod (2) and the sleeve (330); and wherein the elastic ring is configured to surround at least one of the plunger rod (2) and the sleeve (330).

## Patentansprüche

1. Unteranordnung für eine Arzneimittelabgabevorrichtung, die Unteranordnung umfassend:
- eine Kolbenstange (2), umfassend:
∘ einen rohrförmigen Körper (20), der sich entlang einer Längsachse (L) zwischen einem geschlossenen Ende (22) und einem offenen Ende (21) erstreckt; und
∘ einen Pfeifeneingang (23; 23') an einer rohrförmigen Wand des rohrförmigen Körpers (20); wobei der Pfeifeneingang (23; 23') zwischen dem offenen Ende (21) des rohrförmigen Körpers (20) und dem geschlossenen Ende (22) des rohrförmigen Körpers (20) positioniert ist; wobei der rohrförmige Körper (20) der Kolbenstange (2) mindestens eine geneigte Oberfläche (23a; 23a') umfasst, die relativ zu der Längsachse (L) abgewinkelt ist und sich von dem Pfeifeneingang (23; 23') in der Richtung der Längsachse (L) erstreckt;
- ein Kraftpaket, das in der Lage ist, einen Luftstrom zu erzeugen, der in den rohrförmigen Körper (20) strömt.

2. Unteranordnung nach Anspruch 1, wobei der rohrförmige Körper (20) der Kolbenstange (2) eine erste geneigte Oberfläche (23a) und eine zweite geneigte Oberfläche (23b) umfasst; wobei die erste geneigte Oberfläche (23a) relativ zu der Längsachse (L) abgewinkelt ist und sich von dem Pfeifeneingang (23) zu dem offenen Ende (21) des rohrförmigen Körpers (20) hin erstreckt; und wobei die zweite geneigte Oberfläche (23b) relativ zu der Längsachse (L) abgewinkelt ist und sich von dem Pfeifeneingang (23) zu dem geschlossenen Ende (22) des rohrförmigen Körpers (20) hin erstreckt.

3. Unteranordnung nach einem der vorstehenden Ansprüche, umfassend eine Blockiereinheit (6), die innerhalb des rohrförmigen Körpers (20) der Kolbenstange (2) angeordnet ist; wobei die Blockiereinheit (6) entlang der Längsachse (L) zwischen einer blockierten Position, in der die Blockiereinheit (6) mit dem Pfeifeneingang (23) relativ zu der Längsachse (L) radial ausgerichtet ist, und einer Freigabeposition, in der die Blockiereinheit (6) mit der Pfeifeneingang (23) relativ zu der Längsachse (L) radial falsch ausgerichtet ist, bewegbar ist.

4. Unteranordnung nach Anspruch 3, wobei die Blockiereinheit (6) einen Blockierkörper (60) und ein Vorspannelement (61) umfasst und wobei das Vorspannelement (61) zwischen dem geschlossenen Ende (22) des rohrförmigen Körpers (20) und dem Blockierkörper (60) positioniert ist.

5. Unteranordnung nach einem der Ansprüche 1 bis 4; wobei die Unteranordnung umfasst
- ein Gehäuse (10), das sich entlang einer Längsachse (L) zwischen einem proximalen Ende und einem distalen Ende erstreckt; und
- eine Antriebsanordnung (3), die konfiguriert ist, um mit einem Auslöser der Arzneimittelabgabevorrichtung verbunden zu sein; wobei die Antriebsanordnung und der Auslöser ein Teil des Kraftpakets sind;
wobei die Antriebsanordnung (3) mit dem Gehäuse verbunden und mit der Kolbenstange (2) verbunden ist.

6. Unteranordnung nach Anspruch 5, wobei die Antriebsanordnung (3) einen Gasbehälter (31), eine Ventileinheit (32) und eine Hülse (330) umfasst; wobei die Ventileinheit (32) ein Ventil umfasst; wobei das Ventil einen Lufteinlass und einen Luftauslass umfasst; wobei der Lufteinlass mit dem Gasbehälter (31) verbunden ist; wobei sich die Hülse (330) entlang der Längsachse (L) zwischen einer ersten Öffnung (330a) und einer zweiten Öffnung (330b) erstreckt; wobei die erste Öffnung (330a) der Hülse (330) mit dem offenen Ende (21) des rohrförmigen Körpers (20) der Kolbenstange (2) verbunden ist; wobei die zweite Öffnung (330b) der Hülse (330) mit dem Luftauslass des Ventils (320) gasdicht verbunden ist; und wobei der Gasbehälter (31) relativ zu dem Lufteinlass des Ventils (320) zwischen einer Gasfreigabeposition und einer Gasabdichtungsposition axial bewegbar ist.

7. Unteranordnung nach Anspruch 6, wobei die Unteranordnung einen elastischen Ring (4) umfasst, der zwischen dem rohrförmigen Körper (20) der Kolbenstange (2) und der Hülse (330) angeordnet ist; und wobei der elastische Ring konfiguriert ist, um mindestens eine von der Kolbenstange (2) und der Hülse (330) zu umgeben.

## Revendications

1. Sous-ensemble destiné à un dispositif d'administration de médicament, le sous-ensemble comprenant :
- une tige de piston (2) comprenant :
∘ un corps tubulaire (20) s'étendant le long d'un axe longitudinal (L) entre une extrémité fermée (22) et une extrémité ouverte (21) ; et
∘ un orifice de sifflet (23 ; 23') sur une paroi tubulaire du corps tubulaire (20) ; dans lequel l'orifice de sifflet (23 ; 23') est positionné entre l'extrémité ouverte (21) du corps tubulaire (20) et l'extrémité fermée (22) du corps tubulaire (20) ; dans lequel le corps tubulaire (20) de la tige de piston (2) comprend au moins une surface inclinée (23a ; 23a') formant un angle par rapport à l'axe longitudinal (L) et s'étendant depuis l'orifice de sifflet (23 ; 23') dans la direction de l'axe longitudinal (L) ;
- un bloc d'alimentation apte à générer un flux d'air s'écoulant dans le corps tubulaire (20).

2. Sous-ensemble selon la revendication 1, dans lequel le corps tubulaire (20) de la tige de piston (2) comprend une première surface inclinée (23a) et une seconde surface inclinée (23b) ; dans lequel la première surface inclinée (23a) forme un angle par rapport à l'axe longitudinal (L) et s'étend depuis l'orifice de sifflet (23) vers l'extrémité ouverte (21) du corps tubulaire (20) ; et dans lequel la seconde surface inclinée (23b) est inclinée par rapport à l'axe longitudinal (L) et s'étend depuis l'orifice de sifflet (23) vers l'extrémité fermée (22) du corps tubulaire (20).

3. Sous-ensemble selon l'une quelconque des revendications précédentes, comprenant une unité de blocage (6) agencée au sein du corps tubulaire (20) de la tige de piston (2) ; dans lequel l'unité de blocage (6) est mobile le long de l'axe longitudinal (L) entre une position bloquée où l'unité de blocage (6) est alignée radialement avec l'orifice de sifflet (23) par rapport à l'axe longitudinal (L) et une position de libération où l'unité de blocage (6) est désalignée radialement avec l'orifice de sifflet (23) par rapport à l'axe longitudinal (L).

4. Sous-ensemble selon la revendication 3, dans lequel l'unité de blocage (6) comprend un corps de blocage (60) et un élément de sollicitation (61), et dans lequel l'élément de sollicitation (61) est positionné entre l'extrémité fermée (22) du corps tubulaire (20) et le corps de blocage (60).

5. Sous-ensemble selon l'une quelconque des revendications 1 à 4 ; dans lequel le sous-ensemble comprend
- un boîtier (10) s'étendant le long d'un axe longitudinal (L) entre une extrémité proximale et une extrémité distale ; et
- un ensemble d'entraînement (3) configuré pour être connecté à un déclencheur du dispositif d'administration de médicament ; l'ensemble d'entraînement et le déclencheur faisant partie du bloc d'alimentation ;
dans lequel l'ensemble d'entraînement (3) est relié au boîtier et relié à la tige de piston (2).

6. Sous-ensemble selon la revendication 5, dans lequel l'ensemble d'entraînement (3) comprend une cartouche de gaz (31), une unité de valve (32) et un manchon (330) ; dans lequel l'unité de valve (32) comprend une valve ; dans lequel la valve comprend une entrée d'air et une sortie d'air ; dans lequel l'entrée d'air est raccordée à la cartouche de gaz (31) ;dans lequel le manchon (330) s'étend le long de l'axe longitudinal (L) entre une première ouverture (330a) et une seconde ouverture (330b) ; dans lequel la première ouverture (330a) du manchon (330) est reliée à l'extrémité ouverte (21) du corps tubulaire (20) de la tige de piston (2) ; dans lequel la seconde ouverture (330b) du manchon (330) est reliée de manière étanche aux gaz à la sortie d'air de la valve (320) ; et dans lequel la cartouche de gaz (31) est mobile axialement par rapport à l'entrée d'air de la valve (320) entre une position de libération de gaz et une position étanche aux gaz.

7. Sous-ensemble selon la revendication 6, dans lequel le sous-ensemble comprend une bague élastique (4) agencée entre le corps tubulaire (20) de la tige de piston (2) et le manchon (330) ; et dans lequel la bague élastique est conçue pour entourer au moins l'un parmi la tige de piston (2) et le manchon (330).
